# EUROPEAN PATENT APPLICATION

(11) **EP 2 995 256 A1**
(43) Date of publication of application: **16.03.2016**
(21) Application number: 15183938.8
(22) Date of filing: 04.09.2015
(51) Int. Cl.: A61B 8/00, H01B 7/08, H01R 31/06

(54) **CABLE ASSEMBLY FOR CONVERTING A CONSECUTIVE SIGNALING ARRANGEMENT TO AN INTERLEAVED SIGNALING ARRANGEMENT**

(30) Priority: 10.09.2014 US 201414482560
(71) Applicant: Tyco Electronics Corporation, Berwyn, PA 19312 (US)
(72) Inventor: Schroder, Edwin W., Wilsonville, OR Oregon 97070 (US)
(74) Representative: Johnstone, Douglas Ian

(57) **Abstract**

A cable assembly (300) includes a first group of wires (200a) and a second group of wires (200b). Wires in the groups are arranged side-by-side. At respective first ends (210a, 210b), the wires within the groups are held in place relative to one another so that the wires are separated by a first distance (230), and at respective second ends (205a, 205b), the wires within the groups are held in place relative to one another so that the wires are separated by a second distance (225) that is greater than the first distance (230). The first group of wires (200a) is overlaid on the second group of wires (200b) such that at first ends (210a, 210b), the wires of the respective groups (200a, 200b) are consecutively arranged and at second ends (205a, 205b), the wires of the respective groups (200a, 200b) are interleaved with one another.

## Description

### BACKGROUND

The present invention relates generally to a cabling arrangement for an ultrasound system. More specifically, the present invention relates to ribbon cable that facilitates coupling an ultrasound processor with a consecutive signaling arrangement to a probe that has an interleaved signaling arrangement.

Many medical devices include a base unit and a remote unit where the remote unit communicates information to and from the base unit. The base unit then processes information communicated from the remote unit and provides diagnostic information, reports, and the like. In some arrangements, a cable that includes a group of electrical wires couples the remote unit to the base unit. For example, signals may be communicated between an ultrasound probe and an ultrasound processor over a cable that includes hundreds of wires.

The wires within the cable may be provided in the form of a ribbon cable where conductors are arranged next to one another, side-by-side, and held together by a ribbonizing material, such as plastic. For ultrasound systems, the respective ends of the ribbon cable are connected to the ultrasound probe and the ultrasound processor. Connection of the probe and processor is relatively easy when the pin out arrangements on the probe and processor match.

When operating an ultrasound system in CW Doppler mode, the pins on the probe and processor may be configured so that half the pins are used for sending information to the transducers of the probe and the other half of the pins are used for receiving information from the transducers within the probe. In some ultrasound systems, this is accomplished by arranged the pins on the processor and probe so that a first group of adjacent pins is used to transmit transducer signals and a second group of adjacent pins is used to receive transducer signals. For example, pins 1-8 on both the probe and the processor may be used for sending transducer information, and pins 9-16 may be used for receiving transducer information.

However, in other systems, the pin out arrangement of the probe and the processor do not match. For example, instead of having the grouped arrangement described above, the pin out on the probe may be configured so that the function (i.e., receiving and sending) alternates between odd and even-numbered pins. For example, pins one and three on the probe may be used for sending signals to transducers numbered one and two of the probe. Pins two and four may be used for receiving signals from the transducers numbered one and two, and so on. When the pin outs on the probe and processor do not match, an operator typically has to peel individual wires out of the ribbon at one end or the other and re-arrange the wires so that the signals are communicated correctly.

### SUMMARY

In one aspect, a cable assembly includes a first group of wires arranged side-by-side. At a first end, the wires are held in place relative to one another so that the wires are separated by a first distance; and at a second end, the wires are held in place relative to one another so that the wires are separated by a second distance that is greater than the first distance. The cable includes a second group of wires arranged side-by-side. At a first end, the second group of wires are held in place relative to one another so that they are separated by a third distance; and at a second end, the wires are held in place relative to one another so that they are separated by a fourth distance that is greater than the third distance. The first group of wires is overlaid or overlays on the second group of wires such that the first end of the first group of wires is adjacent to the first end of the second group of wires, and each wire of the second end of the first group of wires is interleaved with a wire of the second end of the second group of wires.

In a second aspect, a method for manufacturing a cable includes arranging a first group of wires side-by-side so that at a first end, the wires are separated by a first distance; and at a second end, the wires are separated by a second distance that is greater than the first distance. A ribbonizing material is applied over the first end and the second end of the first group of wires to hold the wires in place. The method also includes arranging a second group of wires side-by-side so that at a first end, the wires are separated by a third distance; and at a second end, the wires are separated by a fourth distance that is greater than the third distance. A ribbonizing material is applied over the first end and the second end of the second group of wires to hold the wires in place. The first group of wires is overlaid on the second group of wires such that the first end of the first group of wires is adjacent the first end of the second group of wires, and each wire of the second end of the first group of wires is interleaved with a wire of the second end of the second group of wires.

In a third aspect, an ultrasound system includes an ultrasound probe, an ultrasound processor, and a cable assembly for communicating signals between the ultrasound probe and the ultrasound processor. The ultrasound probe has a first group of electrical contacts for sending transducer signals, and a second group of electrical contacts interleaved with the first group of electrical contacts for receiving transducer signals. The ultrasound processor has a first group of electrical contacts for sending transducer signals, and a second group of electrical contacts adjacent to the first group of electrical contacts for receiving transducer signals. The cable includes a first group of wires arranged side-by-side. At a first end, the wires are held in place relative to one another so that the wires are separated by a first distance; and at a second end, the wires are held in place relative to one another so that the wires are separated by a second distance that is greater than the first distance. The cable includes a second group of wires arranged side-by-side. At a first end, the second group of wires are held in place relative to one another so that they are separated by a third distance; and at a second end, the wires are held in place relative to one another so that they are separated by a fourth distance that is greater than the third distance. The first group of wires is overlaid on the second group of wires such that the first end of the first group of wires is adjacent to the first end of the second group of wires, and each wire of the second end of the first group of wires is interleaved with a wire of the second end of the second group of wires. The first ends of the first group of wires preferably lie on a line which constitutes an extension of a line on which the first ends of the second group of wires lie.

### BRIEF DESCRIPTION OF THE DRAWINGS

Fig. 1 illustrates an ultrasound system that includes a probe with an interleaved signaling arrangement and a processor with a consecutive signaling arrangement.
Fig. 2 illustrates first and second groups of wires.
Figs. 3A and 3B illustrate assembly of the first and second groups of wires into a cable assembly.
Fig. 4 illustrates a top view of the cable assembly.

### DETAILED DESCRIPTION

A cable assembly that overcomes the problems above is disclosed in detail below. Generally, the cable assembly includes a first group of wires and a second group of wires. At first ends of the respective groups of wires, the wires are spaced apart by a first distance. At second ends of the respective groups of wires, the wires are spaced apart by a second distance that is about double the first distance. The second group of wires is overlaid on top of the second group of wires so that, at the second ends, the respective wires of the groups are interleaved, while at the first end, the respective wires of the groups are arranged in a consecutive arrangement.

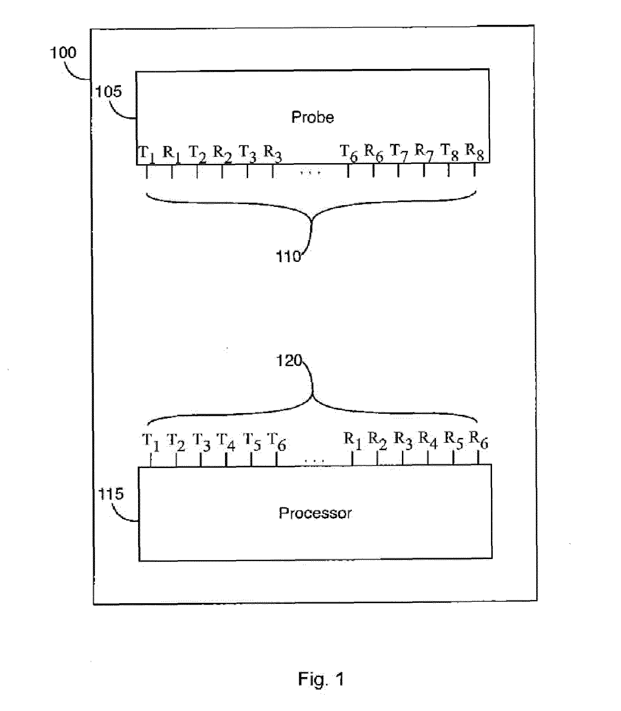

Fig. 1 illustrates an exemplary ultrasound system 100 that may include an ultrasound processor 115 and a probe 105. The probe 105 may include a number of transducers (not shown) configured to transmit an ultrasonic wave and to receive a corresponding echo of the ultrasonic wave. The probe 105 also includes a group of pins 110 arranged in an odd/even or interleaved relationship, where odd-numbered pins are used to transmit signals to respective transducers, and even-numbered pins are used to receive signals from respective transducers. Each transducer is operatively coupled to a pair of pins 110 on the probe. For example, a first transducer is coupled to pins T₁ and R₁, a second transducer is coupled to pins T₂ and R₂, and so on. While only a few pins are illustrated, it should be understood that, in practice, the number of pins might be much larger.

The processor 115 may correspond to any system capable of communicating transducer signals to and from a probe 105 for generating images associated with transducer signals. The processor 115 includes a group of pins 120 configured to send and receive information to corresponding pins 110 of the probe. As illustrated, the pins 120 may be arranged so that all the pins for transmitting transducer signals are grouped together on one side, and all the pins for receiving transducer signals from the probe are grouped together on the other side. In operation, pins 120 on the processor 115 are wired or coupled to like-named pins 110 on the probe. For example, pin T₁ on the processor 115 is wired or coupled to pin T₁ on the probe 105. The number of pins shown is only exemplary and, in practice, might be much larger.

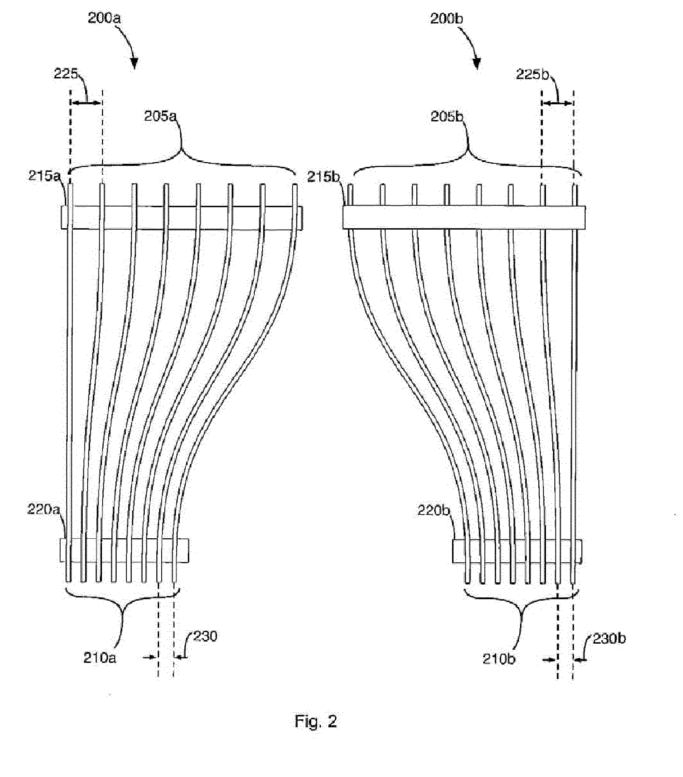

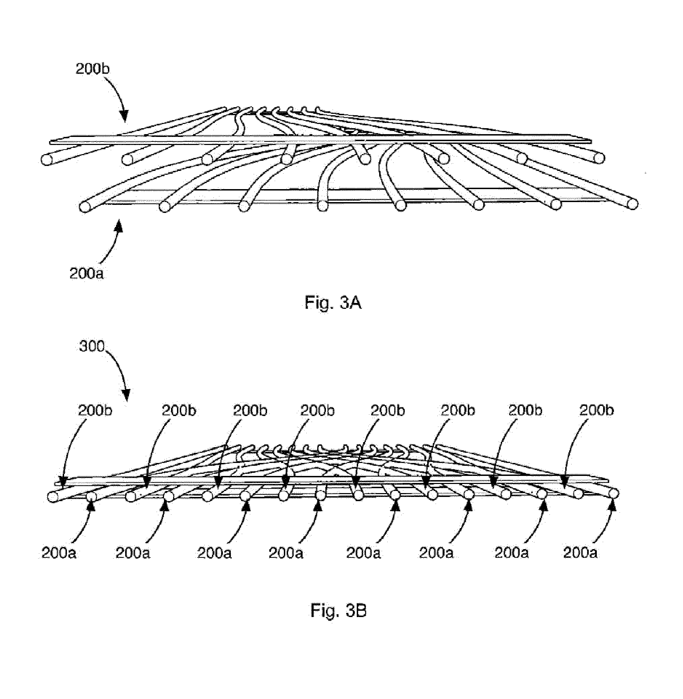

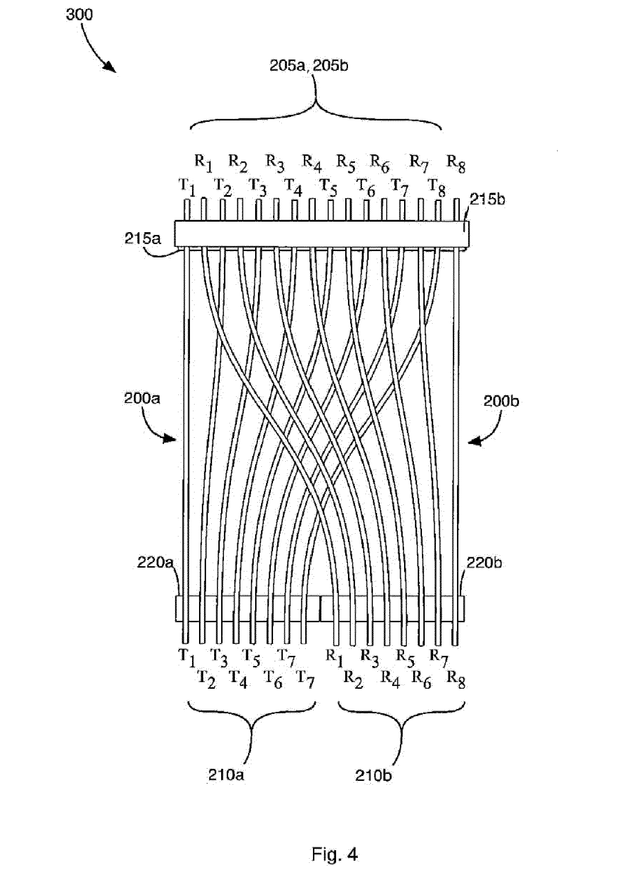

Fig. 2 illustrates portions of an exemplary cable assembly 300 (See Fig. 3B and 4) for coupling the pins 120 of the processor 115 to the pins 110 of the probe 105. The cable assembly 300 includes a first group of wires 200a arranged side-by-side, and a second group of wires 200b arranged side-by-side. In an exemplary implementation, each wire may correspond to a coaxial wire that includes at least a center conductor and a shield. For example, the coaxial wire may be a micro coaxial wire that has diameter of about 0.03556 cm (0.014 inches). A different wire, which has a different number of conductors and/or configuration, may be utilized.

At the first end 210a, the wires of the first group of wires 200a may be spaced apart by a first distance 230 that may correspond roughly to the diameter of each wire so that adjacent wires touch one another. For example, the wires may be spaced apart by about 0.04318 cm (0.017 inches). The wires may be spaced apart by a different distance. At the second end 205a, the wires may be spaced apart by a second distance 225 that is greater than the first distance 230. For example, the second distance 225 between the wires at the second end 205 may correspond to roughly 2x the diameter of each wire, or about 0.07112 cm (0.028 inches). The spacing between the wires at the first end 210b and the second end 205b of the second group of wires 200b may be similarly configured. At a first end 210b, the second group of wires 200b are held in place relative to one another so that they are separated by a third distance 230b; and at a second end 205b, the wires are held in place relative to one another so that they are separated by a fourth distance 225b that is greater than the third distance 230b.

In some implementations, a ribbonizing material 220a, 220b may be applied near respective first ends 210a, 210b of the first and second groups of wires 200a, 200b to hold the wires at the first ends 210a, 210b together. A ribbonizing material 215a, 215b may also be applied near respective second ends 205a, 205b of the first and second groups of wires 200a, 200b to hold the wires at the second ends 205a, 205b together. The ribbonizing material 215a, 215b and 220a, 220b may correspond to an adhesive-backed material, such as polyester film, that may have with a width of between about 2.54 cm (1 inch) and 7.62 cm (3 inches). In alternative implementations, the ribbonizing material may extend the length of the wires 200a, 200b. For example, the ribbonizing material may be applied to the wires 200a, 200b during an extrusion process or other manufacturing processes for ribbonizing a group of wires.

The ribbonizing material 215a at the second end 205a of the first group of wires 200a may be disposed along a bottom or first side of the group of wires 200a, and the ribbonizing material 215b at the second end 205b of the second group of wires 200b may be disposed along a top or opposite second side of the group of wires 200b, as illustrated in Fig. 2. The opposite arrangement is also possible. The ribbonizing material 220a, 220b at the respective first ends 210a, 210b of the first and second groups of wires 200a, 200b may be disposed on either side of the respective groups of wires 200a, 200b.

In operation, the first and second groups of wires 200a, 200b are arranged in the manner described above. As illustrated in Fig. 3A, the second group of wires 200b may be brought over the first group of wires 200a. Next the second group of wires 200b is lowered on to the first group of wires 200a so the ends of the wires are substantially aligned with one another, as illustrated in Fig. 3B, to provide the assembled cable 300. In some implementations, the ribbonizing material 215a, 215b secures the second ends 205a, 205b of the first and second groups of wires 200a, 200b in this configuration. For example, an adhesive on the ribbonizing material 215a, 215b may secure the second ends 205a, 205b in this configuration.

As can be seen in Fig. 4, the cable assembly 300 overcomes the problems described above by converting a consecutive signaling arrangement to an odd/even or interleaved signaling arrangement. For example, the first end 210a of the first group of wires 200a may be coupled to the transmit pins of the ultrasound processor 115 described above, and the first end 210b of the second group of wires 200b may be coupled to the receive pins. The interleaved second ends 205a, 205b of the first and second groups of wires 200a, 200b may be coupled to the interleaved transmit and receive pins of the ultrasound probe 105. This arrangement advantageously does away with the step of peeling apart wires, which is required in known cables.

While the cable assembly 300 has been described with reference to certain embodiments, it will be understood by those skilled in the art that various changes may be made and equivalents may be substituted without departing from the scope of the claims of the application. Various modifications may be made to adapt a particular situation or material to the teachings disclosed above without departing from the scope of the claims. Therefore, the claims should not be construed as being limited to any one of the particular embodiments disclosed, but to any embodiments that fall within the scope of the claims.

## Claims

1. A cable assembly (300) comprising:
a first plurality of wires (200a) arranged side-by-side, wherein at a first end (210a), the wires are held in place relative to one another so that at the first end (210a), the wires are separated by a first distance (230), and at a second end (205a), the wires are held in place relative to one another so that at the second end (205a), the wires are separated by a second distance (225) that is greater than the first distance (230);
a second plurality of wires (200b) arranged side-by-side, wherein at a first end (210b), the second plurality of wires (200b) are held in place relative to one another so that at the first end (210b), the wires are separated by a third distance (230b), and at a second end (205b), the second plurality of wires (200b) are held in place relative to one another so that at the second end (205b), the wires are separated by a fourth distance (225b) that is greater than the third distance (230b),
wherein the first plurality of wires (200a) is overlaid on the second plurality of wires (200b) such that the first end (210a) of the first plurality of wires (200a) is adjacent to the first end (210b) of the second plurality of wires (200b), and each wire of the second end (205a) of the first plurality of wires (200a) is interleaved with a wire of the second end (205b) of the second plurality of wires (200b).

2. The cable assembly (300) according to claim 1, wherein the second distance (225) between the wires of the second end (205a) of the first plurality of wires (200a) is greater than or equal to the diameter of a wire of the second plurality of wires (200b).

3. The cable assembly (300) according to any preceding claim, wherein the first plurality of wires (200a) are held in place at the second end (205a) via a ribbonizing material (215a) that runs along a bottom side of the first plurality of wires (200a), and the second plurality of wires (200b) are held in place at the second end (205b) via a ribbonizing material (215b) that runs along a top side of the second plurality of wires (200b).

4. The cable assembly (300) according to claim 3, wherein the first plurality of wires (200a) are held in place at the first end (210a) via a ribbonizing material (220a) that runs along either a top or a bottom side of the first plurality of wires (200a), and the second plurality of wires (200b) are held in place at the first end (210b) via a ribbonizing material (220b) that runs along either a top or a bottom side of the first plurality of wires (200a).

5. The cable assembly (300) according to claim 3 or 4, wherein the ribbonizing material (215, 215b, 220a, 220b) corresponds to an adhesive-backed material.

6. The cable assembly (300) according to any preceding claim, wherein the first end (210a) of the first plurality of wires (200a) is configured to be coupled to a first group of electrical contacts (120, T₁ ... T₆) for sending transducer signals of an ultrasound processor (115), and the first end (210b) of the second plurality of wires (200b) is configured to be coupled to a second group of electrical contacts (120, R₁...R₆) for receiving transducer signals of the ultrasound processor (115).

7. The cable assembly (300) according to claim 6, wherein the second end (205a) of the first plurality of wires (200a) is configured to be coupled to a first group of electrical contacts (110, T₁...T₈) for receiving transducer signals of the ultrasound probe (105), and the second end (205b) of the second plurality of wires (200b) is configured to be coupled to a second group of electrical contacts (110, R₁...R₈) for sending transducer signals of the ultrasound probe (105).

8. The cable assembly (300) according to any preceding claim, wherein each wire of the first and second plurality of wires (210a, 210b) corresponds to a coaxial wire that includes at least a center conductor and a shield.

9. A method for manufacturing a cable assembly (300) comprising:
arranging a first plurality of wires (200a) side-by-side so that at a first end (210a), the wires are separated by a first distance (230), and at a second end (205a), the wires are separated by a second distance (225) that is greater than the first distance (230);
applying a ribbonizing material (220a, 215a) over the first end (210a) and the second end (205a) of the first plurality of wires (200a) to hold the wires in place;
arranging a second plurality of wires (200b) side-by-side so that at a first end (210b), the wires are separated by a third distance (230b), and at a second end (205b), the wires are separated by a fourth distance (225b) that is greater than the third distance (230b);
applying a ribbonizing material (220b, 215b) over the first end (210b) and the second end (205b) of the second plurality of wires (200b) to hold the wires in place;
overlaying the first plurality of wires (200a) on the second plurality of wires (200b) such that the first end (210a) of the first plurality of wires (200a) is adjacent to the first end (210b) of the second plurality of wires (200b), and each wire of the second end (205a) of the first plurality of wires (200a) is interleaved with a wire of the second end (205b) of the second plurality of wires (200b).

10. The method according to claim 9, wherein the ribbonizing material (215a) that holds the first plurality of wires (200a) in place at the second end (205a) runs along a bottom side of the first plurality of wires (200a), and the ribbonizing material (215b) that holds the second plurality of wires (200b) in place at the second end (205b) runs along a top of the first plurality of wires (200a).

11. The method according to claim 10, wherein the ribbonizing material (220a) that holds the first plurality of wires (200a) in place at the first end (210a) runs along either a top or a bottom side of the first plurality of wires (200a), and the ribbonizing material (220b) that holds the second plurality of wires (200b) in place at the first end (210b) runs along either a top or a bottom side of the second plurality of wires (200b).

12. The method according to claim 9, 10 or 11, further comprising coupling the first end (210a) of the first plurality of wires (200a) to a first group of electrical contacts (120, T₁...T₆) for sending transducer signals of the ultrasound processor (115), and the first end (210b) of the second plurality of wires (200b) to a second group of electrical contacts (120, R₁...R₆) for receiving transducer signals of the ultrasound processor (115),

13. The method according to claim 12, further comprising coupling the second end (205a) of the first plurality of wires (200a) to a first group of electrical contacts (110, T₁...T₈) for receiving transducer signals of the ultrasound probe (105), and coupling the second end (205b) of the second plurality of wires (200b) to a second group of electrical contacts (110, R₁...R₈) for sending transducer signals of the ultrasound probe (105).

14. An ultrasound system (100) comprising:
an ultrasound probe (105) having a first group of electrical contacts (110, T₁...T₈) for sending transducer signals, and a second group of electrical contacts (110, R₁...R₈) interleaved with the first group of electrical contacts (110, T₁...T₈) for receiving transducer signals;
an ultrasound processor (115) having a first group of electrical contacts (120, T₁...T₆) for sending transducer signals, and a second group of electrical contacts (120, R₁...R₆) adjacent to the first group of electrical contacts (120, T₁...T₆), for receiving transducer signals; and
a cable assembly (300) according to any one of claims 1 to 8 for communicating signals between the ultrasound probe (105) and the ultrasound processor (115).
